# EUROPEAN PATENT APPLICATION

(11) **EP 4 527 934 A1**
(43) Date of publication of application: **26.03.2025**
(21) Application number: 23807907.3
(22) Date of filing: 17.05.2023
(51) Int. Cl.: C12N 15/87, A61K 48/00, A61K 47/69

(54) **GENE DELIVERY SYSTEM BASED ON METAL NANOPARTICLE-NUCLEIC ACID CONJUGATE**

(30) Priority: 17.05.2022 KR 20220059957; 16.05.2023 KR 20230063360
(71) Applicant: NES BIOTECHNOLOGY CO., LTD., Seoul 06974 (KR)
(72) Inventor: LEE, Kangseok, Gwangju-si, Gyeonggi-do 12765 (KR); BAE, Jeehyeon, Gwangju-si, Gyeonggi-do 12765 (KR); YEOM, Jihyun, Yangju-si, Gyeonggi-do 11426 (KR); SIM, Sehoon, Suwon-si, Gyeonggi-do 16493 (KR); KIM, Hongman, Suwon-si, Gyeonggi-do 16438 (KR); SONG, Wooseok, Seoul 05698 (KR); RYU, Minkyung, Seoul 07364 (KR); JOO, Minju, Seoul 03638 (KR); LHEE, Sangmoon, Seoul 05229 (KR)
(74) Representative: Dehns
(86) International application number: PCT/KR2023/006697
(87) International publication number: WO 2023/224388

(57) **Abstract**

The present invention relates to a gene carrier including a nucleic acid molecule containing a gene of interest conjugated to the surface of a metal nanoparticle, which is delivered into cells and expressed, a use thereof, a gene expression method using the same, and a method of preparing the same.

## Description

### [Technical Field]

The present invention relates to a gene carrier including a metal nanoparticle and a double-stranded nucleic acid molecule conjugated to the nanoparticle surface, a gene expression method using the same, and a method of preparing the same.

### [Background Art]

Nanoparticles are particles with nanoscale sizes, which have a variety of physical and chemical properties due to their small size and high surface area. Gold nanoparticles, which are the most widely used nanoparticles, generate surface plasmon resonance (SPR) by absorption and scattering in the visible light range depending on their size and shape, so they can be used for detection and imaging based on fluorescent labeling, and can also be used to deliver biomaterials such as DNA, RNA, proteins, and antibodies, and various drugs due to easy introduction of a surface functional group, and their high bioaffinity and high stability.

Technologies, such as cell therapeutics, are being developed to deliver genetic materials into cells to produce antigens or proteins for treatment or prevention, and particularly, vaccine development technology has grown significantly in the wake of the recent pandemic, leading to the development of various gene vaccines such as DNA vaccines, mRNA vaccines, and viral vaccines.

DNA is the simplest form of genetic material and can be easily genetically modified to shorten the development period for therapeutics such as vaccines. In addition, compared to other vaccine candidate materials such as viruses, proteins, and RNA, DNA is very economical in terms of establishment of production facilities and production costs, and due to its excellent stability, it is easy to store and distribute. However, DNA vaccines need to be delivered to the nucleus of a cell to produce mRNA directly within the nucleus. DNA introduced into the nucleus can continuously produce mRNA and steadily produce antigenic proteins, but there is a concern that it may cause side effects such as an innate immune response because other genetic traits are injected into the cell nucleus in the body. In addition, when delivered by a plasmid, DNA may cause side effects such as mutation in the body by delivering a bacterial gene, in addition to an antigen. Accordingly, it is necessary to study carriers and delivery techniques to safely deliver DNA.

### [Disclosure]

### [Technical Problem]

Therefore, as a result of making extensive efforts to develop a nucleic acid delivery technology to efficiently deliver a nucleic acid molecule, particularly, double-stranded DNA (dsDNA), which can be delivered into a cell and expressed alone, to the nucleus within the cell, the present inventors have completed the present invention relating to a carrier system that can deliver a nucleic acid molecule containing a gene of interest into a cell by directly attaching it onto the surface of a metal nanoparticle through a covalent bond, a gene expression method using the same, and a method of preparing the carrier system.

Accordingly, the present invention is directed to providing a gene carrier, which includes a metal nanoparticle; and a nucleic acid molecule containing one or more genes of interest, which is conjugated to the metal nanoparticle, delivered into a cell, and expressed.

The present invention is also directed to providing a pharmaceutical composition including the gene carrier.

The present invention is also directed to providing a composition for detecting a target material, which includes the gene carrier.

The present invention is also directed to providing a composition for delivering a gene of interest into a cell, which includes the gene carrier.

The present invention is also directed to providing a method of preparing a gene carrier, which includes modifying the surface of a metal nanoparticle by treating the metal nanoparticle with an acidic solution; and conjugating a nucleic acid molecule containing one or more genes of interest, which is delivered into cells and expressed, to the surface of the metal nanoparticle.

The present invention is also directed to providing a method of expressing a gene of interest through a nucleic acid molecule that is expressed alone in cells by conjugating the nucleic acid molecule to the surface of a metal nanoparticle.

### [Technical Solution]

To address the above-described objects, the present invention provides a gene carrier, which includes a metal nanoparticle; and a nucleic acid molecule containing one or more genes of interest, which is conjugated to the metal nanoparticle, delivered into a cell, and expressed.

The present invention also provides a pharmaceutical composition, which includes the gene carrier.

The present invention also provides a composition for detecting a target material, which includes the gene carrier.

The present invention also provides a composition for delivering a gene of interest into a cell, which includes the gene carrier.

The present invention also provides a method of preparing a gene carrier, which includes modifying the surface of a metal nanoparticle by treating the metal nanoparticle with an acidic solution; and conjugating a nucleic acid molecule containing one or more genes of interest, which are delivered into cells and expressed, to the surface of the metal nanoparticle.

The present invention also provides a method of expressing a gene of interest through a nucleic acid molecule expressed alone in cells by conjugating it to the surface of a metal nanoparticle.

Hereinafter, the present invention will be described in detail.

**In** one aspect, the present invention relates to a gene carrier, which includes a metal nanoparticle; and a nucleic acid molecule containing one or more genes of interest, which is conjugated to the metal nanoparticle, delivered into a cell, and expressed.

The gene carrier of the present invention includes a metal nanoparticle. The metal nanoparticle has a nanoscale diameter, for example, 5 to 500 nm, and more preferably, 10 to 200 nm, but the present invention is not limited thereto. For nanoparticles with this size, it is easy to prepare a stable form of particles and easy to control their size during the preparation process. Moreover, when the metal nanoparticle has a size of 500 nm or more, not only does it lose its properties as a nanoparticle, but also the bonding between a metal surface and a functional group is weakened, making it difficult to prepare a carrier using the nanoparticles. In addition, the metal nanoparticle may preferably be a gold nanoparticle, and unlike heavy metals such as manganese, aluminum, cadmium, lead, mercury, cobalt, nickel, and beryllium, the gold nanoparticle is harmless to the human body and has high biocompatibility.

In one embodiment, the gold nanoparticle used in the present invention may be prepared as follows: HAuCl₄ is used as a gold source, sodium citrate is used as a reducing agent to reduce HAuCl₄, thereby preparing a gold nanoparticle. In this case, the size of the gold nanoparticle can be controlled by changing the amount of citrate added. That is, as the amount of citrated added increases, the size of the metal nanoparticle decreases due to more nucleation.

The metal nanoparticle of the present invention has a nucleic acid molecule containing a gene of interest bound to its surface. The nucleic acid molecule is conjugated to the surface of the metal nanoparticle to deliver a gene of interest to be introduced into cells and is expressed alone.

The nucleic acid molecule is not limited in its type. In the present invention, the nucleic acid molecule refers to a compound with a structure consisting of a base, a sugar, and phosphoric acid, which are linked by phosphodiester bonds and includes naturally-occurring oligonucleotides such as 2'-deoxyribonucleic acid (hereinafter, referred to as "DNA") or ribonucleic acid (hereinafter, referred to as "RNA") and nucleic acids including a modified sugar residue, a modified phosphate residue, or a modified nucleobase. The modification of a sugar residue includes replacement of a ribose ring with a hexose, cyclopentyl or cyclohexyl ring. Alternatively, the D-ribose ring of a naturally-occurring nucleic acid may be replaced with an L-ribose ring, or the β-anomer of a naturally-occurring nucleic acid may be replaced with an α-anomer. The nucleic acid molecule may also include one or more abasic moieties. Modified phosphate moieties may also include phosphorothioates, phosphorodithioates, methylphosphonates, and methyl phosphates. Such nucleic acid analogs are known in the art. A nucleic acid molecule containing two or more of the above mixtures may be produced from, for example, a mixture of deoxyribo- or ribonucleosides, particularly, the mixture of a deoxyribonucleoside and a 2'-O-substiuted ribonucleoside such as 2'-O-methyl ribonucleoside or 2'-O-methoxyethyl ribonucleoside.

More specifically, the nucleic acid molecule may be selected from DNA, RNA or DNA/RNA molecules, and even more specifically, double-stranded DNA. The double-stranded DNA may include cDNA, gDNA, plasmid DNA, and PCR DNA, which can be expressed alone. In one embodiment, the double-stranded DNA is bound to a gold nanoparticle in a double-stranded state, allowing the double-stranded DNA to be delivered into a cell. This is different from single-stranded DNA conjugated to a gold nanoparticle, which then enters the cell and hybridizes with a complementary strand.

In addition, the nucleic acid molecule may include one or more genes of interest, which may be expressed alone after entering cells. The term "gene of interest" used herein includes any nucleic acid that has therapeutic, diagnostic, and/or preventive effect(s) and/or induces a desired biological and/or pharmacological effect(s), or a nucleic acid that encodes a functional peptide or polypeptide of interest (protein)(native or modified peptide/protein).

"Expressed alone" used herein means that a gene of interest contained in the nucleic acid molecule is transcribed and/or translated alone without being incorporated into the genome of an injected cell. In one example, to be expressed alone, the nucleic acid molecule may include one or more promoters, open reading frames, or terminators, and more preferably, one or more promoters that are operably linked to the gene of interest, but the present invention is not limited thereto. A promoter sequence is a eukaryotic promoter, often derived or cleaved from a virus, and thus, the promoter may be a proopiomelanocortin promoter (POMC), an adenovirus promoter, a baculovirus promoter, a CMV promoter, a parvovirus promoter, a herpes virus promoter, a fox virus promoter, an adeno-associated virus promoter, a Semliki Forest Virus promoter, a SV40 promoter, a vaccinia virus promoter, or a retrovirus promoter. Examples of the promoters include a human simplex virus thymidine kinase (HSV TK or miniTK) promoter, a cauliflower mosaic virus (CaMV) 35S promoter, a human cytomegalovirus CMV promoter (miniCMV), CMV53 (upstream GC box-added minCMV), a Simian virus 40 promoter (minSV40), MLP (the -38 to +6 region of an adenovirus major late promoter), minP (a synthetic promoter consisting of a TATA box and a transcription initiation site; produced by Promega), pJB42CAT5 (human junB gene-derived promoter), YB_TATA, and a super core promoter 1 (SCP1) promoter. Some promoters (occasionally called a "core promoter") are described in the document (Ede et al., ACS Synth Biol. 2016 May 20; 5(5): 395-404).

The term "operably disposed," "operably binding," and "operably linked" mean that a promoter (and/or an enhancer) is at a precise functional location and orientation relative to a nucleic acid sequence to control the transcription initiation and expression of that nucleic acid. An enhancer is "operably linked" to a promoter when it is in the correct functional location and orientation to increase the transcriptional activity of the promoter.

In one embodiment, the nucleic acid molecule further includes a polyadenylation (poly(A)) sequence. The poly(A) sequence causes proper polyadenylation of a nucleic acid of interest (transcriptome). The representative examples of poly(A) sequence include SV40 poly(A) and/or bovine growth hormone poly(A), known to be convenient and/or function well in various target cells.

In one embodiment, the nucleic acid molecule further includes a transcription termination sequence. "Termination sequence" or "termination factor" consists of a DNA sequence involved in termination specific to an RNA transcript by an RNA polymerase.

In addition, the nucleic acid molecule may produce a transcription and/or translation product(s) through an expression process including transcription and/or translation, but the present invention is not limited thereto. The transcription and/or translation product(s) may include, for example, mRNA, non-coding RNA, a protein, an antigen, or an antibody, but the present invention is not limited thereto.

The gene carrier of the present invention has a nucleic acid molecule conjugated to the surface of a gold nanoparticle. The nucleic acid molecule includes one or more functionalities for conjugation to the gold nanoparticle surface. The functionality is not limited in type, and may be a thiol group or an amine group, which may be included in one or more residues of the nucleic acid molecule to be delivered. In one embodiments of the present invention, the nucleic acid molecule includes one or more thiolated residues, through which it directly binds to the surface of the gold nanoparticle. The binding does not include a separate spacer or linker. The thiolated residue may be one or more bases included at the 3' end, the 5' end, or in the base sequence of the nucleic acid molecule.

In addition, one or more nucleic acid molecules may be bound to the gold nanoparticle surface, and for expression, 1 to 20 nucleic acid molecules may be bound to the surface of the gold nanoparticle, but the present invention is not limited thereto. In addition, the length of the bound nucleic acid molecule is at least 100 bp, 200 bp, or 300 bp, but the present invention is not limited thereto. In addition, in some cases, the nucleic acid molecule is more than 30 nt long. In another embodiment, the nucleic acid molecule is more than 35 nt. In still another embodiment, the length is at least 40 nt. In yet another embodiment, the length is at least 45 nt. In yet another embodiment, the length is at least 55 nt. In yet another embodiment, the length is at least 50 nt. In yet another embodiment, the length is at least 60 nt. In yet another embodiment, the length is at least 80 nt. In yet another embodiment, the length is at least 90 nt. In yet another embodiment, the length is at least 100 nt. In yet another embodiment, the length is at least 120 nt. In yet another embodiment, the length is at least 140 nt. In yet another embodiment, the length is at least 160 nt. In yet another embodiment, the length is at least 180 nt. In yet another embodiment, the length is at least 200 nt. In yet another embodiment, the length is at least 250 nt. In yet another embodiment, the length is at least 300 nt. In yet another embodiment, the length is at least 350 nt. In yet another embodiment, the length is at least 400 nt. In yet another embodiment, the length is at least 450 nt. In yet another embodiment, the length is at least 500 nt. In yet another embodiment, the length is at least 600 nt. In yet another embodiment, the length is at least 700 nt. In yet another embodiment, the length is at least 800 nt. In yet another embodiment, the length is at least 900 nt. In yet another embodiment, the length is at least 1000 nt. In yet another embodiment, the length is at least 1100 nt. In yet another embodiment, the length is at least 1200 nt. In yet another embodiment, the length is at least 1300 nt. In yet another embodiment, the length is at least 1400 nt. In yet another embodiment, the length is at least 1500 nt. In yet another embodiment, the length is at least 1600 nt. In yet another embodiment, the length is at least 1800 nt. In yet another embodiment, the length is at least 2000 nt. In yet another embodiment, the length is at least 2500 nt. In yet another embodiment, the length is at least 3000 nt. In yet another embodiment, the length is at least 4000 nt. In yet another embodiment, the length is at least 5000 nt, or more than 5000 nt.

In another aspect of the present invention, the present invention relates to a pharmaceutical composition, which includes the gene carrier that includes a gold nanoparticle and double-stranded DNA. The pharmaceutical composition is for the prevention, improvement, or treatment of a disease, and while not limited to its type, depending on the type and expression product of double-stranded DNA conjugated to the gold nanoparticle, its use may vary.

The pharmaceutical composition may be a cell/gene therapeutic, such as a cell therapeutic, a genetically-modified cell therapeutic, a gene therapeutic, or an RNA therapeutic, and the cell/gene therapeutic may include a vaccine, an antibiotic, and an anticancer agent.

The cell therapeutic refers to a drug used for therapeutic, diagnostic, and preventive purposes by a series of actions, such as growing, selecting, or altering the biological properties of living autologous, allogenic, or xenogeneic cells *in vitro* to restore the tissue and function of the cells, and is sometimes referred to as a gene modified cell therapeutic when modifying a gene within a cell.

In addition, the gene therapeutic is a drug prepared to treat or prevent a genetic defect by correcting a defective gene or adding a new function to a cell by introducing normal and therapeutic genes into cells of a patient using genetic manipulation, such as genetic recombination.

Moreover, the RNA therapeutic may exhibit the efficacy of a drug while inhibiting the process of producing a protein inducing a disease from a target gene, and include mRNA, RNAi, an antisense oligonucleotide (ASO), and an RNA aptamer.

The pharmaceutical composition according to the present invention may further include appropriate carrier, excipient and diluent, which are conventionally used in the preparation of a pharmaceutical composition. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be formulated in the form of a powder, a granule, a sustained-release granule, an enteric granule, a liquid, an ophthalmic solution, an elixir, an emulsion, a suspension, a spirit, a troche, aromatic water, a lemonade, a tablet, a sustained-release tablet, an enteric tablet, a sublingual tablet, a hard capsule, a soft capsule, a sustained-release capsule, an enteric capsule, a pill, a tincture, a soft extract, a dry extract, a fluid extract, an injection, a capsule, a perfusate, or an external preparation such as a plaster, a lotion, a paste, a spray, an inhalant, a patch, a sterile injection, or an aerosol according to a conventional method, and the external preparation may be formulated in a cream, a gel, a patch, a spray, an ointment, a plaster, a lotion, a liniment, a paste or a cataplasma.

Carriers, excipients, and diluents, which can be included in the pharmaceutical composition according to the present invention, may include lactose, dextrose, sucrose, an oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil.

The pharmaceutical composition according to the present invention may be prepared using a diluent or an excipient such as a filler, a thickening agent, a binder, a wetting agent, a disintegrant, or a surfactant, which is commonly used.

The pharmaceutical composition according to the present invention is administered at a pharmaceutically effective amount. The "pharmaceutically effective amount" used herein refers to an amount sufficient for treating a disease at a reasonable benefit/risk ratio applicable for medical treatment, and an effective dosage may be determined by parameters including the type and severity of a patient's disease, drug activity, sensitivity to a drug, administration time, an administration route and an excretion rate, the duration of treatment and drugs simultaneously used, and other parameters well known in the medical field. The pharmaceutical composition according to the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without side effects, and such a dose may be easily determined by one of ordinary skill in the art.

The pharmaceutical composition of the present invention may be administered to a subject via various routes. All administration routes may be contemplated, and the pharmaceutical composition of the present invention may be administered by, for example, oral administration, subcutaneous injection, intraperitoneal administration, intravenous, intramuscular or intrathecal injection, sublingual administration, buccal administration, rectal insertion, vaginal insertion, ocular administration, ear administration, nasal administration, inhalation, spraying through the mouth or nose, skin administration, or transdermal administration.

The pharmaceutical composition of the present invention is determined according to the type of drug as an active ingredient as well as various related parameters such as a disease to be treated, an administration route, a patient's age, sex or body weight, and the severity of a disease.

In the present invention, "individual" refers to a target subject in need of treatment for a disease, and more specifically, a mammal such as a human or a non-human primate, a mouse, a rat, a dog, a cat, a horse, or a cow.

In the present invention, "administration" refers to the provision of the composition of the present invention to a subject by a suitable method.

In the present invention, "prevention" refers to all actions involved in inhibiting or delaying the onset of a target disease, "treatment" refers to all actions involved in improving or beneficially changing a disease and its metabolic abnormalities by administering the pharmaceutical composition according to the present invention, and "alleviation" refers to all actions involved in reducing parameters related to a target disease, e.g., the severity of symptoms, by administering the composition according to the present invention.

In one aspect of the present invention, the present invention provides a vaccine composition, which includes a gene carrier that includes a metal nanoparticle and a nucleic acid molecule containing a gene of interest. The term "vaccine" is a biological preparation containing an antigen that imparts immunity to a subject, and refers to an immunogenic or antigenic material imparting immunity, which is administered to a person or animal, for example, by injection or oral administration for the prevention of a disease.

The vaccine may be a DNA vaccine. The "DNA vaccine" refers to a vaccine causing an immune response by artificially replicating some of genes of a pathogen or virus and then administering the resultant. The vaccine composition may form immunity against various infectious diseases, genetic diseases, other diseases, or cancer.

The vaccine composition may be injected into a subject in various forms. The "injection" may be performed by any one method selected from the group consisting of subcutaneous, intramuscular, intradermal, intraperitoneal, nasal, oral, transdermal, and oral administrations, and more preferably, any route suitable for the administration of a DNA vaccine, for example, subcutaneous administration, intramuscular injection, intraperitoneal injection, or intravenous injection.

The vaccine composition may include one or more adjuvants to improve or enhance the immune response. Suitable adjuvants may include a peptide, aluminum hydroxide, aluminum phosphate, aluminum oxide, a composition consisting of a mineral oil such as Marcol 52 or a vegetable oil, and one or more emulsifiers, or a surfactant such as lysolecithin, a polyvalent cation, or a polyvalent anion.

In another aspect of the present invention, the present invention provides a composition for diagnosis or detecting a target material, which includes the gene carrier that includes a metal nanoparticle and a nucleic acid molecule containing a gene of interest.

In still another aspect of the present invention, the present invention relates to a composition for delivering a gene of interest into a cell, which includes the metal nanoparticle and a nucleic acid molecule containing a gene of interest.

In yet another aspect of the present invention, the present invention provides a method of preparing a gene carrier, which includes modifying the surface of a metal nanoparticle by treating the metal nanoparticle with an acidic solution; and conjugating a nucleic acid molecule containing one or more genes of interest, which is delivered into cells and expressed, to the surface of the metal nanoparticle.

In yet another aspect of the present invention, the present invention relates to a method of expressing a gene of interest using a nucleic acid molecule expressed alone in cells by conjugating it to the surface of a metal nanoparticle.

### [Advantageous Effects]

The present invention relates to a gene carrier that includes a metal nanoparticle, and a nucleic acid molecule containing a gene of interest conjugated to the surface of the metal nanoparticle, a method of preparing the same, and a use thereof. Here, a nucleic acid molecule containing one or more genes, particularly, a double-stranded DNA-like nucleic acid molecule can directly bind to the surface of a gold nanoparticle through covalent bonds to be delivered into a cell and expressed, thereby enabling stable delivery and expression and utilization thereof as a gene therapeutic, a vaccine, and a composition for diagnosing a disease.

### [Description of Drawings]

FIG. 1 is a schematic diagram of the structure of a nucleic acid molecule containing a gene of interest conjugated to a gold nanoparticle according to one embodiment of the present invention.
FIG. 2 shows the results of confirming the binding efficiency of a nucleic acid molecule conjugated to a gold nanoparticle of the present invention via a thiolated residue.
FIG. 3 shows the results of confirming whether a gene is delivered into a cell according to a thiolated strand ((+), (-)) of double-stranded DNA conjugated to a gold nanoparticle of the present invention.
FIG. 4 illustrates a preparation example for AuNP-dsDNA enabling intracellular delivery and expression of thiolated double-stranded DNA with various lengths by conjugating it to a gold nanoparticle.
FIG. 5 shows the results obtained by conjugating thiolated double-stranded DNA to the surface of a gold nanoparticle after functionalization.
FIG. 6 shows the results of confirming the gene delivery and expression in small animal models of gold nanocarriers in which double-stranded DNA molecules with various lengths of SEQ ID NOs: 1 to 4 are loaded.
FIG. 7 shows the results of functionalizing the luciferase gene (SEQ ID NO: 5) on the surface of a gold nanoparticle.
FIG. 8 is the result that confirms whether a luciferase gene is delivered and expressed in cells using a gold nanocarrier.

### [Modes of the Invention]

Hereinafter, the present invention will be described in detail with reference to examples to help in understanding the present invention. However, examples according to the present invention may be modified in a variety of different forms, and it should not be construed that the scope of the present invention is limited to the following examples. The examples of the present invention are provided to more completely explain the present invention to those of ordinary skill in the art.

### Example 1. Preparation of double-stranded DNA (GFP)-conjugated gold nanoparticle (AuNP-dsDNA)

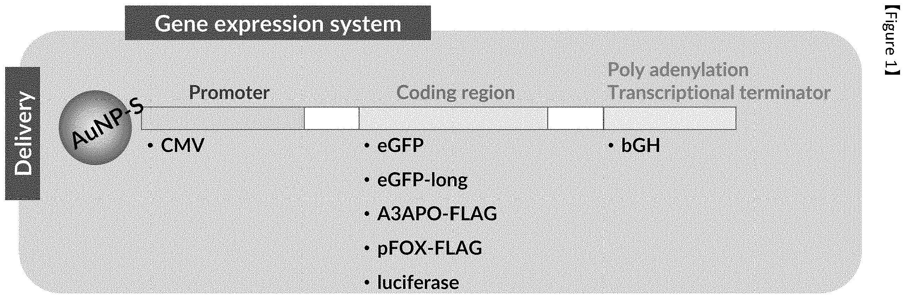

AuNP-dsDNA that enables intracellular delivery and expression by conjugating thiolated double-stranded DNA to be expressed in cells to a gold nanoparticle was prepared by the following steps. The schematic diagram of AuNP-dsDNA is shown in FIG. 1.

### 1-1. Preparation of dsDNA enabling antigen expression in cells

Using plasmid pcDNA3.1, a gene to be delivered included between the CMV promoter and the bGH terminator of the plasmid was cloned. Double-stranded DNA in which the 5' end, the 3' end, or an internal residue was thiolated was synthesized by PCR using thiolated primers.

As the gene to be delivered, the eGFP gene of SEQ ID NO: 1 was cloned by the above-described method, 5' end-thiolated primer sequences in Table 1 below were used to thiolate the 5' end, the 3' end, or an internal residue, and eGFP-thiolated double-stranded DNA was synthesized.

**[Table 1]**

| **Thiolation type** | **Thiolated strand** | | **eGFP sequence** |
|---|---|---|---|
| | **(+)** | **(-)** | |
| **5' end** | - | - | 5' CTTAGGGTTAGGCGTTTTGC ~~ (eGFP expression system) ~~ GTATCCCCACGCGCCCTGTAG 3' |
| | | | 3' GAATCCCAATCCGCAAAACG ~~ (eGFP expression system) ~~ CATAGGGGTGCGCGGGACATC 5' |
| | ○ | - | 5' (Thiolation) CTTAGGGTTAGGCGTTTTGC ~~ (eGFP expression system) ~~ GTATCCCCACGCGCCCTGTAG 3' |
| | | | 3' GAATCCCAATCCGCAAAACG ~~ (eGFP expression system) ~~ CATAGGGGTGCGCGGGACATC 5' |
| | - | ○ | 5' CTTAGGGTTAGGCGTTTTGC ~~ (eGFP expression system) ~~ GTATCCCCACGCGCCCTGTAG 3' |
| | | | 3' GAATCCCAATCCGCAAAACG ~~ (eGFP expression system) ~~ CATAGGGGTGCGCGGACATC (Thiolation) 5' |
| | ○ | ○ | 5' (Thiolation) CTTAGGGTTAGGCGTTTTGC ~~ (eGFP expression system) ~~ GTATCCCCACGCGCCCTGTAG 3' |
| | | | 3' GAATCCCAATCCGCAAAACG ~~ (eGFP expression system) ~~ CATAGGGGTGCGCGGACATC (Thiolation) 5' |
| **Internal residue** | - | ○ | 5' CTTAGGGTTAGGCGTTTTGC ~~ (eGFP expression system) ~~ GTATCCCCACGCGCCCTGTAG 3' |
| | | | 3' GAATCCCAATCCGCAAAACG ~~ (eGFP expression system) ~~ CA*T*A*G*G*GGTGCGCGGACATC 5' |

µL of 1N dithiothreitol (DTT) was added, and reacted at room temperature for 60 minutes. To remove the DTT containing an undesired thiol group molecule, 200 µL of ethyl acetate was added and mixed, and then centrifuged to remove a supernatant, which was repeated three times. Subsequently, thiolated double-stranded DNA was precipitated using an EtOH precipitation method.

### 1-3. Preparation of dsDNA functionalized gold nanoparticle (AuNP-dsDNA)

The thiolated double-stranded DNA, which had been precipitated by pretreatment according to step 1-2, was dissolved in water, and then added to a gold nanoparticle to conjugate by a salt aging method. Specifically, the thiolated double-stranded DNA was added to 7 nM of the gold nanoparticle and sufficiently stirred (AuNP : thiolated double-stranded DNA= 1:40), and NaCl was added to reach a concentration of 0.1M and mixed for 4 hours. Four hours later, NaCl was added to reach a concentration of 0.2M and mixed for 4 hours. Four hours later, NaCl was added to reach a concentration of 0.3M and mixed for 12 hours.

Twelve hours later, the mixture of the thiolated double-stranded DNA and gold nanoparticle was collected by centrifugation at ~10,000 x g for 20 minutes, unreacted double-stranded DNA in the supernatant was removed, which was repeated three times.

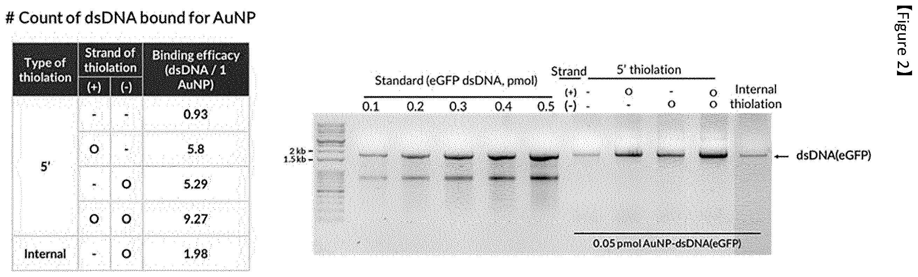

The final AuNP-thiolated double-stranded DNA conjugate (AuNP-thiolated dsDNA) was added to 10 mM sodium phosphate buffer (pH 7.4) containing 0.1 M NaCl and dispersed. The prepared AuNP-thiolated double-stranded DNA conjugate was analyzed by electrophoresis on a 10% acrylamide 8M urea gel, confirming that 1.98 to 9.27 thiolated double-stranded DNA molecules were bound to one gold nanoparticle (FIG. 2).

### Example 2. Confirmation of gene delivery and expression by AuNP-dsDNA (GFP) in small animal models

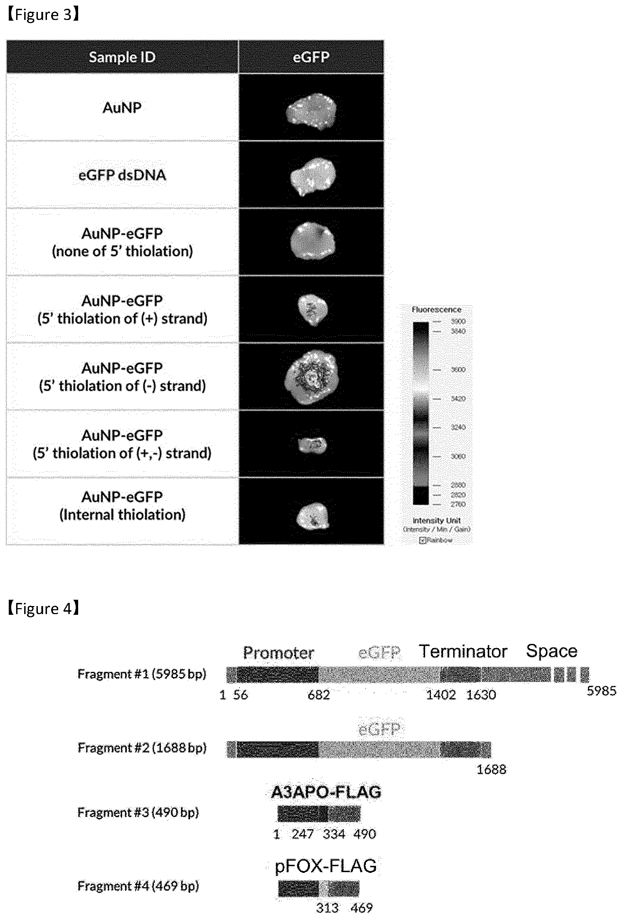

For the convenience of an experiment, xenograft tumor models were used. HeLa cells were injected into a 6-week-old immunodeficient BALB/c-nu/nu mouse (Central Lab Animal Inc, Korea) to cause cervical cancer, and AuNP-thiolated dsDNA was injected into a xenograft tumor. Thirty-six hours later, the xenograft tumor was extracted to observe the expression of the injected GFP using the Fluorescence In Vivo Imaging System (FOBI). As a result, it was confirmed that GFP was expressed in tumors into which the AuNP-thiolated dsDNA, which was thiolated at the 5' end of a (+) strand, a (-) strand, or the (+)/(-) strand or had an internal thiol group, had been injected (FIG. 3).

### Example 3. Preparation of gold nanoparticles to which dsDNAs with different lengths are conjugated

AuNP-dsDNA enabling intracellular delivery and expression were prepared by conjugating thiolated double-stranded DNA with various lengths to gold nanoparticles. The schematic diagram of the AuNP-dsDNA is shown in FIG. 4.

### 3-1. Preparation of double-stranded DNA enabling intracellular expression

Using plasmid pcDNA3.1, eGFPs (SEQ ID NO: 1 and SEQ ID NO: 2), A3APO-FLAG (SEQ ID NO: 3), and pFOX-FLAG (SEQ ID NO: 4) genes, which are included between the CMV promoter and the bGH terminator of the plasmid were cloned. Double-stranded DNA in which one of the 5' end, the 3' end, and an internal residue was thiolated was synthesized through PCR using 5'-thiolated primers.

The synthesized thiolated double-stranded DNA was pretreated in the same manner as Experimental Example 1-2, and the thiolated double-stranded DNA was precipitated using an EtOH precipitation method.

### 3-2 Preparation of dsDNA functionalized gold nanoparticle (AuNP-dsDNA)

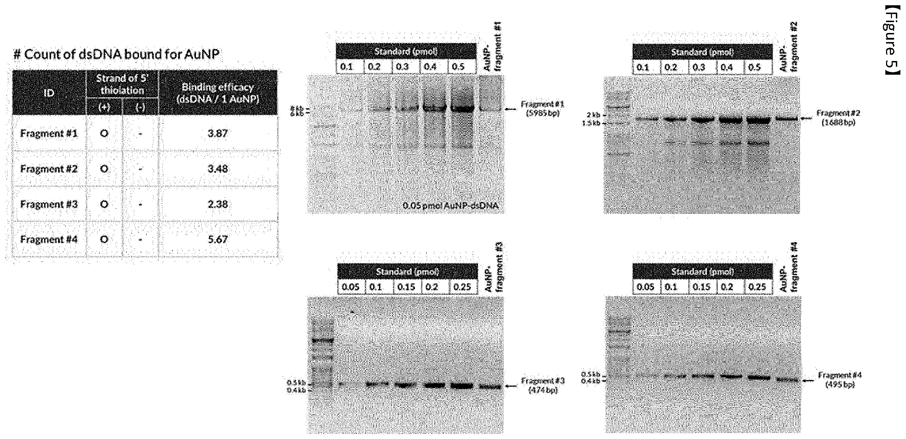

The thiolated double-stranded DNA, which had been synthesized, pretreated and precipitated according to step 3-1, was functionalized on the surface of a gold nanoparticle in the same manner as in step 1-3. The prepared AuNP-thiolated dsDNA conjugate was analyzed by electrophoresis on a 10% acrylamide 8M urea gel, confirming that 2.38 to 5.67 thiolated double-stranded DNA molecules were conjugated to one gold nanoparticle (FIG. 5).

### Example 4. Confirmation of gene delivery and expression of gold nanoparticles in which dsDNAs with various lengths were loaded in small animal models

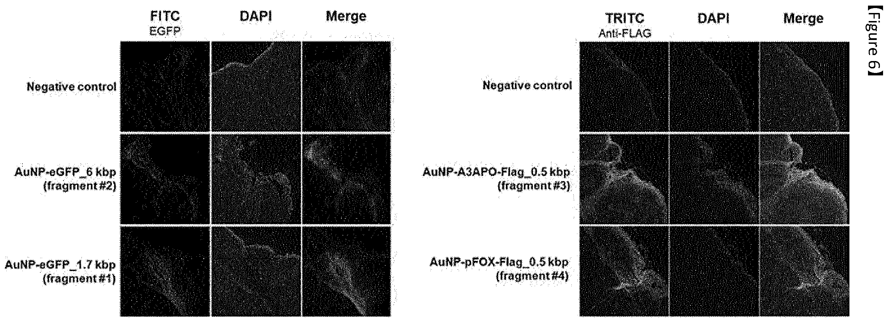

For the convenience of an experiment, xenograft tumor models were used. HeLa cells were injected into a 6-week-old immunodeficient BALB/c-nu/nu mouse (Central Lab Animal Inc, Korea) to cause tumors, and AuNP-thiolated dsDNA conjugated with double-stranded DNA of SEQ ID NOs: 1 to 4 (6 kbp, 1.7 kbp, or 0.5 kbp) was injected around the tumors. Thirty-six hours later, xenograft tumors or gastrocnemius muscles were excised, sections were created by frozen sectioning, and protein expression by the injected dsDNA was observed using immunofluorescence staining. As a result, the protein expression by 6 kbp, 1.7 kbp, or 0.5 kbp dsDNA (SEQ ID NOs: 1 to 4) was confirmed in mouse tumors or gastrocnemius muscles (FIG. 6).

### Example 5. Preparation of gold nanoparticle expressing luciferase

To confirm whether a gold nanoparticle carrier is well expressed in *in vivo* mouse tissue, AuNP-dsDNA that enables intracellular delivery and expression was prepared by conjugating the thiolated double-stranded DNA expressing luciferase to a gold nanoparticle.

### 5-1. Preparation of double-stranded DNA enabling intracellular expression

Using plasmid pcDNA3.1, the luciferase gene (SEQ ID NO: 5) included between the CMV promoter and the bGH terminator of the plasmid was cloned. Double-stranded DNA in which one of the 5' end, the 3' end, and an internal residue was thiolated was synthesized by PCR using thiolated primers.

The thiolated double-stranded DNA synthesized as above was pretreated in the same manner as Experimental Example 1-2, and thiolated double-stranded DNA was precipitated by an EtOH precipitation method.

### 5-2 Preparation of dsDNA functionalized gold nanoparticle (AuNP-dsDNA)

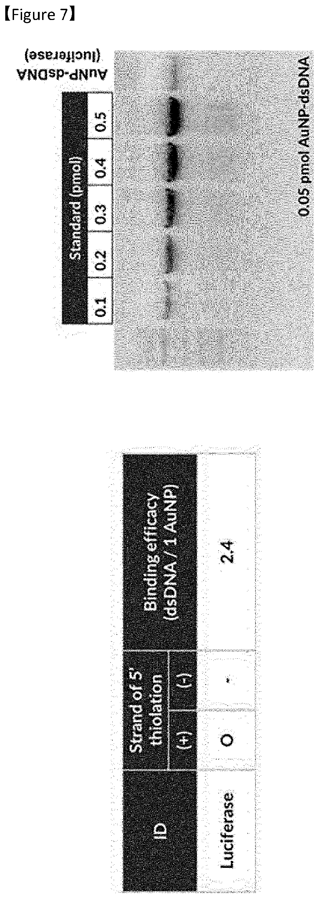

The thiolated double-stranded DNA, which had been precipitated by pretreatment according to step 5-1, was functionalized on the surface of a gold nanoparticle in the same manner as in step 1-3. The prepared AuNP-thiolated dsDNA conjugate was analyzed by electrophoresis on a 10% acrylamide 8M urea gel, confirming that 2.4 thiolated double-stranded DNA molecules were conjugated to one gold nanoparticle (FIG. 7).

### Example 6. Confirmation of gene delivery and expression by AuNP-dsDNA (luciferase) in small animal models

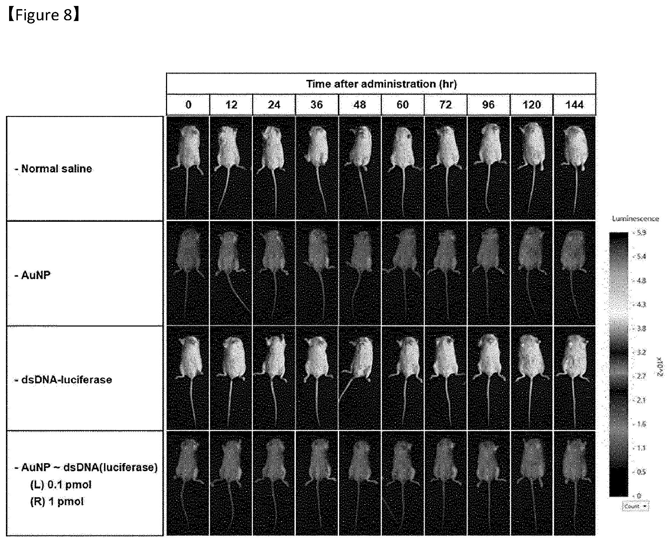

Physiological saline or AuNP-thiolated dsDNA was injected into the gastrocnemius muscles of both sides of a 7-week-old Balb/c mouse, and a corresponding amount of AuNP or luciferase dsDNA was injected as a comparative group. After injection, the mouse was anesthetized using isofluorane every 12 or 24 hours, and D-luciferin was injected intraperitoneally to measure luciferase activity. As a result of imaging with the Luciferase In Vivo Imaging System (LUCI), the enzyme activity of luciferase in the gastrocnemius muscle injected with luciferase dsDNA conjugated to AuNP was confirmed (FIG. 8).

**In** the above, the present invention was described with reference to embodiments. It will be understood by those of ordinary skill in the art that the present invention can be implemented in modified forms without departing from the essential features of the present invention. Therefore, the disclosed embodiments should be considered from a descriptive perspective, rather than a restrictive perspective. The scope of the present invention is shown in the claims rather than the foregoing description, and all differences within the equivalent range thereto will be construed as being included in the present invention.

**In** one aspect of the present invention, the present invention relates to a gene carrier, which includes a metal nanoparticle; and a nucleic acid molecule containing one or more genes of interest, which is conjugated to the metal nanoparticle, delivered into a cell, and expressed.

The nucleic acid molecule includes a promoter operably linked to a gene of interest.

The gene of interest is not replicated in cells.

The gene of interest is not integrated into the genome of the injected cell.

The gene of interest produces a product through transcription and/or translation in cells.

The product produced by transcription and/or translation may be selected from the group consisting of mRNA, non-coding RNA, a protein, an antigen, and an antibody.

The nucleic acid molecule is expressed alone or independently in cells.

The nucleic acid molecule binds to the metal nanoparticle surface via one or more thiolated residues.

One or more thiolated residues may be included at the 3' end or the 5' end of the nucleic acid molecule, and in the base sequence of the nucleic acid molecule.

The metal nanoparticle may have a size of 5 to 500 nm.

The metal nanoparticle may be a gold nanoparticle.

The nucleic acid molecule may be selected from DNA, RNA, or a DNA/RNA molecule.

The DNA is double-stranded DNA.

The nucleic acid molecule may be at least 100 bp, 200 bp, or 300 bp long.

In another aspect of the present invention, the present invention relates to a pharmaceutical composition including the gene carrier.

In still another aspect of the present invention, the present invention relates to a composition for detecting a target material, which includes the gene carrier.

In yet another aspect of the present invention, the present invention relates to a composition for intracellularly delivering a gene of interest, which includes a metal nanoparticle; and a nucleic acid molecule containing one or more genes of interest, which is conjugated to the metal nanoparticle, delivered into a cell, and expressed.

In yet another aspect of the present invention, the present invention relates to a method of preparing a gene carrier, which includes modifying the surface of a metal nanoparticle by treating the metal nanoparticle with an acidic solution; and conjugating a nucleic acid molecule containing one or more genes of interest, which is delivered into cells and expressed, to the surface of the metal nanoparticle.

In yet another aspect of the present invention, the present invention relates to a method of expressing a gene of interest via a nucleic acid molecule expressed alone in cells, by being conjugated to the surface of a metal nanoparticle.

## Claims

1. A gene carrier, comprising:
a metal nanoparticle; and
a nucleic acid molecule containing one or more genes of interest, which is conjugated to the metal nanoparticle, delivered into a cell, and expressed.

2. The gene carrier of claim 1, wherein the nucleic acid molecule includes a promoter operably linked to the gene of interest.

3. The gene carrier of claim 1, wherein the gene of interest is not replicated in cells.

4. The gene carrier of claim 1, wherein the gene of interest is not incorporated into the genome of the injected cell.

5. The gene carrier of claim 1, wherein the gene of interest produces a product through transcription and/or translation in cells.

6. The gene carrier of claim 5, wherein the product produced through transcription and/or translation is one or more selected from the group consisting of mRNA, non-coding RNA, a protein, an antigen, and an antibody.

7. The gene carrier of claim 1, wherein the nucleic acid molecule is expressed alone or independently in cells.

8. The gene carrier of claim 1, wherein the nucleic acid molecule is conjugated to the metal nanoparticle surface via one or more thiolated residues.

9. The gene carrier of claim 8, wherein one or more of the thiolated residues are included at the 3' end or the 5' end of the nucleic acid molecule, or within the base sequence thereof.

10. The gene carrier of claim 1, wherein the metal nanoparticle has a size of 5 to 500 nm.

11. The gene carrier of claim 1, wherein the metal nanoparticle is a gold nanoparticle.

12. The gene carrier of claim 1, wherein the nucleic acid molecule is selected from DNA, RNA, and a DNA/RNA molecule.

13. The gene carrier of claim 12, wherein the DNA is double-stranded DNA.

14. The gene carrier of claim 1, wherein the nucleic acid molecule is at least 100 bp, 200 bp, or 300 bp long.

15. A pharmaceutical composition comprising the gene carrier of claim 1.

16. A composition for detecting a target material comprising the gene carrier of claim 1.

17. A composition for delivering a gene of interest into cells, comprising:
a metal nanoparticle; and
a nucleic acid molecule containing one or more genes of interest, which is conjugated to the metal nanoparticle, delivered into cells, and expressed.

18. A method of preparing a gene carrier, comprising:
modifying the surface of a metal nanoparticle by treating the metal nanoparticle with an acidic solution; and
conjugating a nucleic acid molecule containing one or more genes of interest, which is delivered into cells and expressed, to the surface of the metal nanoparticle.

19. A method of expressing a gene of interest through a nucleic acid molecule expressed alone in cells by conjugating the nucleic acid molecule to the surface of a metal nanoparticle.
